# EUROPEAN PATENT APPLICATION

(11) **EP 1 967 975 A2**
(43) Date of publication of application: **10.09.2008**
(21) Application number: 08305048.4
(22) Date of filing: 05.03.2008
(51) Int. Cl.: G06F 19/00

(54) **Measured vital data preserving and providing system for healthcare**

(30) Priority: 05.03.2007 JP 2007054937
(71) Applicant: TANITA CORPORATION, Tokyo 174-8630 (JP)
(72) Inventor: Sato, Katsutoshi, Tokyo 174-8630 (JP); Sakai, Yasunobu, Tokyo 174-8630 (JP); Hasegawa, Hiroki, Tokyo 174-8630 (JP)
(74) Representative: Michelet, Alain

(57) **Abstract**

**Object**

It is to provide a measured vital data preserving and providing system for healthcare, making it unnecessary to have an independent data base which requires a lot of time and a lot of cost for its preparation, and on the other hand, enabling to construct a unique website for healthcare in a short time and at a low cost.

**Means for achieving the object**

A data providing side server 30 comprises a measured vital data DB50 composed of measured vital data records including user's IDs and user's measured vital data, and an API library 60 for processing the measured vital data DB50. In response to responding to a request which is sent from a data utilizing side server 20 to utilize a selected function of the API library 60 by specifying the user's ID, a responding section 32 of the data providing side server 30 can process measured vital data records recorded in the measured vital data DB50 by means of the selected function of the API library 60 so as to a predetermined response to the data utilizing side server 20.

## Description

### Field of the Invention

The present invention relates to a measured biological or living body or vital data preserving and providing system for healthcare, including a data providing side server and a data utilizing side server, which are connected via a network.

### Description of the Related Art

Recently, metabolic syndrome, which increases the risk of onset of cerebral apoplexy caused by arterial sclerosis and other diseases, has become a social issue. On the other hand, people take a growing interest in their health day by day, and therefore, are anxious to have a support scheme for easy self-healthcare is required. Accordingly, various healthcare systems are being developed, in which vital data of users measured by a weight scale, a body fat meter or the like is collected in a server of a healthcare service provider so that vital data thus collected can be utilized for healthcare.

### Disclosure of Invention

### Problems that the Invention is intended to solve

The above described healthcare systems of the healthcare service providers comprise a database to preserve and manage the measured vital data, and a website is configured depending on a business development at the healthcare service provider, in order to provide various healthcare services in response to a use's request.

However, if an independent database is prepared for each healthcare service provider, a problem is encountered in which not only a lot of time is required for a database construction, but also a lot of cost is needed for maintenance and management of the database. If each healthcare service provider attempts to construct a unique website, another problem is also encountered that a lot of time and cost are necessary for creating programs and contents.

Accordingly, an object of the present invention is to solve the aforementioned problems and to provide a measured vital data preserving and providing system for healthcare, making it unnecessary for each healthcare service provider to have an independent data base which requires a lot of time and a lot of cost for its preparation, and on the other hand, enabling each healthcare service provider to construct a unique website for healthcare in order to provide unique healthcare services.

### Means for solving the problems

According to a first aspect of the present invention, there is provided a measured vital data preserving and providing system for healthcare, comprising a data providing side server and a data utilizing side server, which are connected via a network, the data utilizing side server being configured to respond to a user's request so as to provide a service obtained by utilizing the data preserved in the data providing side server, the data providing side server comprising a data record section for recording data records including an identifier concerning a user and data associated with the identifier, a library of a plurality of functions capable of processing the data records recorded in the data record section, and a responding means for responding to a request which is sent from the data utilizing side server and which identifies a selected identifier and requests to utilize a function selected from the plurality of functions of the library, so as to provide to the data utilizing side server a predetermined response obtained by processing the data records recorded in the data record section by use of the selected function of the library.

In the above mentioned measured vital data preserving and providing system for healthcare in accordance with the present invention, the data providing side server includes a service providing means responding to a request from a user to provide a predetermined service based on the predetermined response supplied from the responding means.

In the above mentioned measured vital data preserving and providing system for healthcare in accordance with the present invention, the identifiers included in the data records are user's identifiers, and the data included in the data records is a vital data of users measured by a vital data measuring device.

In the above mentioned measured vital data preserving and providing system for healthcare in accordance with the present invention, at least one function of the plurality of functions of the library is a function for retrieving the data records preserved in the data record section, with reference to the identifier designated in the request sent from the data utilizing side server.

In the above mentioned measured vital data preserving and providing system for healthcare in accordance with the present invention, the identifier designated in the request sent from the data utilizing side server may be an identifier enciphered in a predetermined manner.

According to a second aspect of the present invention, there is provided a data providing program for a data providing side server to provide data via a network, the data providing side server being connected to a data utilizing side server via the network, the data providing side server comprising a data record section for recording data records including an identifier concerning a user and data associated with the identifier, and a library of a plurality of functions capable of processing the data records recorded in the data record section, the data providing program enabling a computer of the data providing side server to function as a responding means for responding to a request which is sent from the data utilizing side server and which identifies a selected identifier and requests to utilize a function selected from the plurality of functions of the library, so as to provide to the data utilizing side server a predetermined response obtained by processing the data records recorded in the data record section by use of the selected function of the library.

In the above mentioned data providing program in accordance with the present invention, the identifiers included in the data records are user's identifiers, and the data included in the data records is a vital data of users measured by a vital data measuring device.

The above mentioned data providing program in accordance with the present invention can decipher the identifier enciphered in a predetermined manner, designated in the request sent from the data utilizing side server.

According to a third aspect of the present invention, there is provided a computer-readable recording medium that records any one of the above mentioned data providing programs in accordance with the present invention.

According to a fourth aspect of the present invention, there is provided a data providing method for providing data from a data providing side server to a data utilizing side server via a network, the data providing side server comprising a data record section for recording data records including an identifier concerning a user and data associated with the identifier, and a library of a plurality of functions capable of processing the data records recorded in the data record section, the data utilizing side server responding to a user's request so as to provide a service obtained by utilizing the data preserved in the data providing side server, the method including the steps of :
causing the data utilizing side server to generate a request which identifies a selected identifier and requests to utilize a function selected from the plurality of functions of the library, to the data providing side server. and
causing the data providing side server to provide to the data utilizing side server via the network a predetermined response obtained by processing the data records recorded in the data record section and designated by the identifier included in the request, by use of the function selected by the request, of the library of the data providing side server.

### Advantages of Invention

In the measured vital data preserving and providing system for healthcare and the others in accordance with the present invention, the data providing side server comprising a measured vital data record section for recording data records including a user's identifier and a user's measured vital data, and an API (application programming interface) library including a plurality of functions capable of processing the data records recorded in the measured vital data record section. A responding means of the data providing side server can respond to a request which is sent from the data utilizing side server and which identifies the user's identifier and utilizes a function selected from the API library, so as to provide to the data utilizing side server a predetermined response obtained by processing the measured vital data records recorded in the measured vital data record section by use of the selected function of the API library. Thus, since the data utilizing side server can utilize the measured vital data records supplied from the data providing side server, the data utilizing side server no longer needs to independently construct a data base of measured vital data with a lot of time and a lot of cost. Since the API library of the data providing side server can be utilized, it is possible to specialize in processing the data obtained and in displaying the outcome of the data processing, with the result that a unique website for healthcare can be advantageously constructed with a short time and a low cost.

### Brief Description of Drawings

Fig. 1 shows a measured vital data preserving and providing system 1 for healthcare, which is an Embodiment 1 of the present invention;
Fig. 2(A) and 2(B) show examples of measured vital data recorded in a database DB50;
Fig. 3 shows in table form an example of functions defined in an API library 60.
Fig. 4 is a flowchart illustrating various steps of the data providing program or the data providing method in accordance with the present invention;.
Fig. 5 shows a measured vital data preserving and providing system 2 for healthcare, which is an Embodiment 2 of the present invention; and
Fig. 6 is a block diagram of an internal circuit 70 of a computer of the data providing side server 30 or the data utilizing side server 20, which executes the computer programs of the present invention to implement each embodiment.

### Detailed Description of Preferred Embodiments

### First Embodiment

Now, embodiments of the present invention will be described in details with reference to the attached drawings.

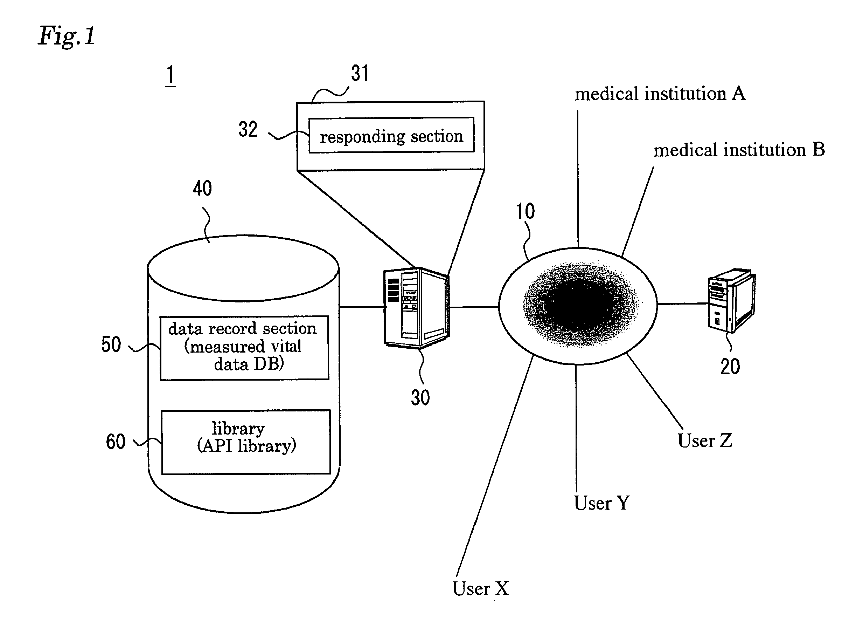

Fig. 1 shows a measured vital data preserving and providing system 1 for healthcare, which is an Embodiment 1 of the present invention. In Fig. 1, Reference Numeral 10 designates a network such as Internet or the like. Reference Numeral 30 indicates a data providing side server connected to the network 10. Reference Numeral 20 denotes a data utilizing side server connected to the network 10. Therefore, the data providing side server 30 and the data utilizing side server 20 are interconnected via the network 10. Furthermore, medical institutions A, B, C, ... and users X, Y, Z, ... are or can be connected to the network 10. In the drawing, only one data utilizing side server 20 is shown, but it is to be understood that at least one data utilizing side server is provided for each healthcare service provider. Accordingly, in some cases, a medical institution would serve as a healthcare service provider, and in other cases, a healthcare service provider may be a sport club or an esthetic salon or alternatively, an on-line healthcare service provider independently of the medical institution. In addition, the data utilizing side server 20 is in no way limited to the healthcare service provider, but may be a research organization, since a huge amount of measured vital data accumulated in the data providing side server 30 is highly valuable to medical or other researches. For example, if a user supplying his or her measured vital data to the data providing side server 30 previously gives an authorization for utilization by research organizations, it is preferred that the accumulated measured vital data can be utilized by those research organizations. For the purpose of enabling such utilization, a user's identifier is preferably constructed to include a code indicating whether or not the authorization is given for utilization by research organizations. In such a case, the measured vital data whose user's identifier gives the authorization for utilization by research organizations, is extracted, and then, is subjected to a primary processing based on the object of a research so that a statistical data excluding any information enabling to identify an individual, such as the number, the residential area, or the age distribution of users having the measured vital data value within a designated range, can be provided to the research organization (which can be deemed as the healthcare service provider.

Reference Numeral 40 designates a storage device such as a disk storage in the data providing side server 30. The storage device 40 comprises a database (data record section) 50 composed of records (data records) which include an arbitrary identifier and data associated to the identifier. The data is preferably a user's living body or vital data measured by a living body measuring equipment (not shown) such as a body fat meter or a sphygmomanometer or the like. In this case, the identifier is preferably a user's identifier of each user (user's ID). Hereinafter, the data record is referred to as measured vital data record, and the data record section 50 is referred to as a database of measured vital data (measured vital data DB50).

As shown in Fig. 1, the storage device 40 includes a library 60 of various functions in a form available from a program namely an application, in order to for example retrieve the measured vital data DB50 or further perform a primary processing for the retrieved data. This library 60 is preferably API (Application Programming Interface), which is a set of functions or programs having a function to perform various retrievals on the measured vital data DB50 and various primary processing for the retrieved data. Hereinafter, the library 60 is referred to as an API library 60.

In Fig. 1, Reference Numeral 31 designates a function block showing the functions of the data providing side server 30. A responding section (responding means) 32 is shown as the corresponding functions. For example, the responding section 32 responds to a request which is sent from the data utilizing side server 20 and which designates the user's ID and at least one function of the various functions of the API library 60 so as to utilize the at least one function, and processes the measured vital data designated by the user's ID, in the measured vital data records stored in the measured vital data DB50, by means of the designated at least one function of the various functions of the API library 60, so as to provide the resultantly obtained measured vital data (namely, a predetermined response) to the data utilizing side server 20.

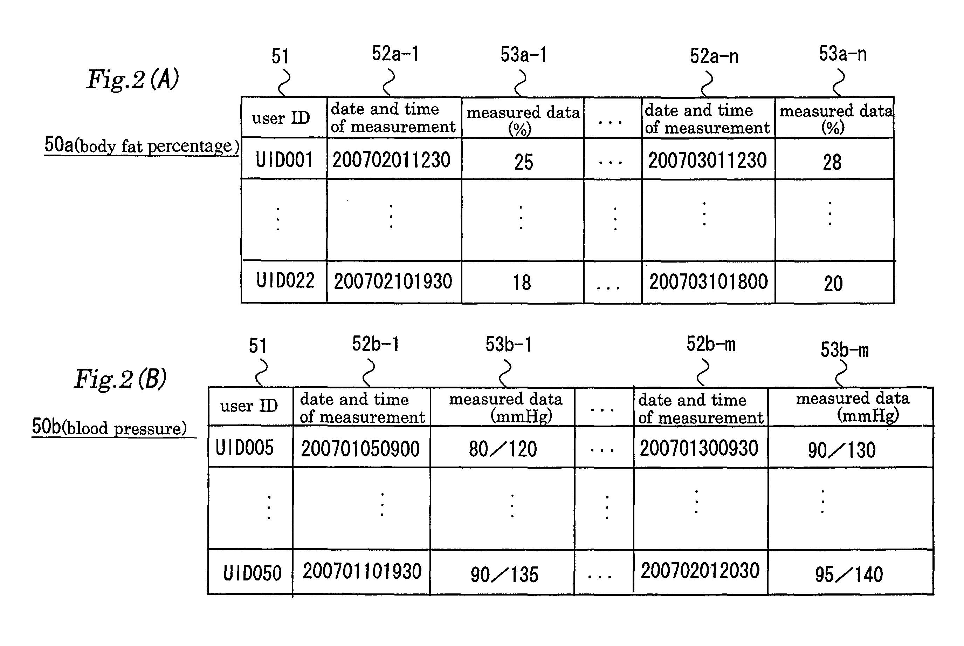

Fig. 2(A) and 2(b) show an example of the measured vital data recorded in the measured vital data DB50. Fig. 2 (A) shows a database 50a composed of measured vital data records regarding a body fat percentage, and Fig. 2 (B) shows a database 50b composed of measured vital data records regarding a blood pressure, as examples.

In Fig. 2 (A), Reference Numeral 51 denotes a user's ID column, and Reference Numeral 52a-1 indicates a measurement date and time column described in the form of YYYYMMDDHHMM (YYYY for year, MM for month, DD for date, HH for hour, MM for minute). Reference Numeral 53a-1 designates a measured data column (unit is shown by %) of the body fat percentage measured at the date and time shown in the measurement date and time column 52a-1, and Reference Numeral 53a-n shows a measured data column (unit is shown by %) of the body fat percentage measured at the date and time shown in the measurement date and time column 52a-n. As exemplified in the first line in Fig. 2(A), the body fat percentage of the user identified by UID001 in the user's ID column 51 and measured at 12:30 o'clock on February 1, 2007 is 25%, and the body fat percentage of the same user measured at 12:30 o'clock on March 1, 2007 is 28%. As seen from Fig. 2 (A), the date and time of measurement and the measured data regarding the body fat percentage are recorded in pairs for each of a plurality of users. Only "n" pairs (the number of measurements) of the data and the measurement date and time are shown in Fig. 2 (A) because of the drawing size, but the number of measurements is not limited to "n".

Also in Fig. 2 (B), Reference Numeral 51 designates a user's ID column, and Reference Numeral 52b-1 indicates a measurement date and time column described in the form of YYYYMMDDHHMM. Reference Numeral 53b-1 denotes a measured data column of the blood pressure (unit is shown by mmHg and expressed in the form of minimal blood pressure / maximum blood pressure) measured at the date and time shown in the measurement date and time column 52b-1. Reference Numeral 53b-m is a measured data column of the blood pressure measured at the date and time shown in the measurement date and time column 52b-m. As exemplified in the first line in Fig. 2 (B), the blood pressures of the user identified by UID005 in the user's ID column 51 measured at 09:00 o'clock on January 5, 2007 are 80 mmHg in minimal and 120 mmHg in maximum, and the blood pressures of the same user measured at 09:30 o'clock on January 1, 2007 are 90 mmHg in minimal and 130 mmHg in maximum. As shown in Fig. 2 (B), the date and time of the measurement and the measured data regarding the blood pressure are recorded in pairs for each of a plurality of users. Only "m" pairs (the number of measurements) of data are shown in Fig. 2 (B) because of the drawing size, but the number of measurements is not limited to "m".

In Fig. 2(A) and 2(b), the measured vital data DB50 is shown as composed of the independent databases 50a and 50b regarding the body fat percentage and the blood pressure, respectively, but this is only an example and the database 50a and 50b may be held as one database.

The above mentioned various measured vital data are collected and preserved in the data providing side server 30 as follows: For example, "measured vital data" measured at the medical institution A, B, C, ··· is combined with information of a measured person, namely, the user's ID and the measurement data and time information, and is supplied from the medical institution A, B, C,··· through the network 10 to the data providing side server 30. Furthermore, "measured vital data" measured at home of the user X, Y, Z, ··· is also combined with information of the measured person, namely, the user's ID and the measurement data and time information, and is supplied from a personal computer at the user's home through the network 10 to the data providing side server 30. In addition, when "vital data" is measured in a sport club or an esthetic salon by the user per se or a staff of the sport club or the esthetic salon, the obtained "measured vital data" is combined with information of a measured person, namely, the user's ID and the measurement data and time information, and is supplied through the network 10 to the data providing side server 30. The data providing side server 30 receiving the data combined with the user's ID, stores the "measured vital data" together with the measurement date and time information in the row of the measured vital data DB50 designated by the user's ID, as shown in Fig. 2(A) and 2(b). In this case, incidentally, the "measured vital data" can be sent to the data utilizing side server 20, and thereafter, the data utilizing side server 20 can sent the received "measured vital data" via the network to the data providing side server 30.

When the user measures the vital data at the user's home, a household weight scale, a household body composition measuring apparatus, a household body fat meter, a household pulsimeter, a household sphygrommanometer, and a household urine sugar meter are used. If the measuring instrument of the type having a function capable of automatically supplying the result of measurement to the data providing side server 30 via Internet is used, or alternatively, if the measuring instrument is associated with an apparatus having such a function, it is possible to efficiently send the result of measurement without no load to the user.

Here, it is to be noted that the "measured vital data" obtained in the medical institutions, the "measured vital data" obtained in the user's homes, and the "measured vital data" obtained in the sport club or the esthetic salon are different in measurement item and in measurement frequency. Since such "measured vital data" of various different measurement items different in measurement frequency are collected and accumulated in the data providing side server 30, it is possible to collect and accumulate a large volume of measured vital data of many measurement items, extremely more than the volume which can be collected by a single healthcare service provider. As a result, each healthcare service provider can effectively utilize the large volume of measured vital data of many measurement items, so as to provide different healthcare services of a high level or reliability or variety. On the other hand, the research organizations could facilitate their research by utilizing the large volume of measured vital data of many measurement items.

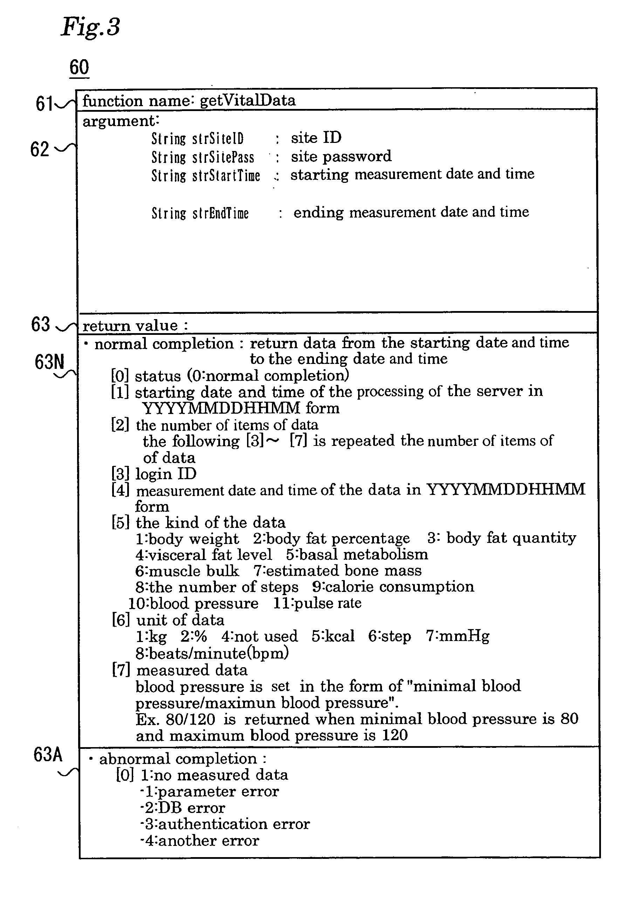

Fig. 3 shows in a table form an example of functions or programs (hereinafter called "function or the like") defined in the API library 60. In Fig. 3, Reference Numeral 61 designates a function name section of the function or the like defined in API library 60 (the function name is "getVitalData"), and Reference Numeral 62 indicates an argument section taken by the function "getVitalData". Reference Numeral 63 shows a return value section returned by the function "getVitalData". Reference Numeral 63N indicates an example of the return value section when the function "getVitalData" is normally completed, and Reference Numeral 63A indicates an example of the return value section when the function "getVitalData" is abnormally completed. The shown example of the function "getVitalData" is to retrieve and return the measured vital data regarding an identified user recorded in the measured vital data DB50 from the starting measurement date and time to the ending measurement date and time.

The identification of a user may be executed using another function or the like based on the user's ID previously sent from the data utilizing side server 20. In a case which will be explained hereinafter, a request for all measured vital data of the user identified by the above mentioned user's ID recorded in measured vital data DB50 from the starting measurement date and time to the ending measurement date and time is a request utilizing a selected function of API library 60 indicating the user's ID, and the corresponding measured vital data constitutes the predetermined response provided to the data utilizing side server 20. Incidentally, it is preferable to use a user's identifier dedicated to communication and a user's identifier which is used in an internal processing of the server. In this case, it is preferred that the user's identifier used in an internal processing of the data providing side server is different from the user's identifier used in an internal processing of the data utilizing side server, and each of both the servers includes a translation table or function for translating between the communication dedicated user's identifier and the internal processing dedicated user's identifier.

As shown in the argument section 62 in Fig. 3, there are four arguments taken by the function "getVitalData". The name of the first argument is "strSiteID" whose data type is a string type, and refers to a site ID which identifies the data utilizing side server 20. The name of the second argument is "strSitePass" whose data type is also the string type, and refers to a site password used to authenticate the data utilizing side server 20. It is preferred to use an IP address of the data utilizing side server 20 as the site password. The name of the third argument is "strStartTime" whose data type is also the string type, and refers to a starting measurement date and time (starting date) expressed in the YYYYMMDDHHMM form. In case of a null character (""), it refers to the top date and time recorded in the measured vital data DB50. The name of the fourth argument is "strEndTime" whose data type is also the string type, and refers to a ending measurement date and time (ending date) expressed in the YYYYMMDDHHMM form. In case of a null character (""), the date and time when this function "getVitalData" is called is applied. As mentioned later, because the return value of the function "getVitalData" includes the starting date and time of the processing of the data providing side server 30 in connection with the outstanding function, a standard way of calling the function "getVitalData" is preferred to designate, as "strStartTime", the starting date and time of the processing of the data providing side server 30 that was returned when the function "getVitalData" was called last time and at the same time to designate a null character as "strEndTime". Thus, the measured vital data successive in the date and time of measurement can be obtained on the side calling this function (the data utilizing side server 20).

Now, the return value returned by the function "getVitalData" will be explained. As shown in the return value section of the normal completion case 63N in Fig. 3, there are eight return values when the function "getVitalData" is normally completed. The first return value (shown by [0]) shows a status. It means the normal completion when the return value is 0. Since the section 63N exemplifies the return value in the normal completion, the returned status is 0. The second return value (shown by [1]) is the starting date and time of the processing of the data providing side server 30, expressed in the YYYYMMDDHHMM form. When any specific value other than the null character is designated as the above described argument "strEndTime", there may be a difference between the designated date and time and the second return value, and therefore, a decision may be needed on the calling side as to whether or not the second return value should be used as "strStartTime" next time. The third return value (shown by [2]) is the number of items of measured vital data ("n" or "m" shown in Fig. 2(A) and 2(B)) returned by this function. This number of items shows the number of sets of measured vital data, each one measured vital data set being composed of the values from the fourth return value to the eighth return value.

The fourth return value (shown by [3]) is the above described user's login ID designated separately. As mentioned above, the user's vital data is measured by a living body measuring equipment (not shown) such as a body fat meter or a sphygmomanometer and is transferred from the measuring equipment via a personal computer (PC) and a network to the data providing side server 30. In this case, since it is necessary to make login in advance from the user side to the data providing side server 30, the above mentioned login ID is used to make this login.

The fifth return value (shown by [4]) is the measurement date and time of the measured vital data in the YYYYMMDDHHMM form. The sixth return value (shown by [5]) is the kind of the measured vital data. The kind of the measured vital data is a body weight in the case of the return value "1", a body fat percentage in the case of the return value "2", a body fat quantity in the case of the return value "3", an visceral fat level in the case of the return value "4", a basal metabolism in the case of the return value "5", a muscle bulk in the case of the return value "6", an estimated bone mass in the case of the return value "7", the number of steps (pedometrical number) in the case of the return value "8", a calorie consumption in the case of the return value "9", a blood pressure in the case of the return value "10", and a pulse rate in the case of the return value "11". Although the eleven kinds of data are shown as the sixth return value in Fig. 3, this is only exemplication and the sixth return value is in no way limited to the eleven kinds mentioned above. The seventh return value (shown by [6]) is a unit of the measured vital data returned as the sixth return value. The unit of the measured vital data is "kg" in the case of the return value "1", "%" in the case of the return value "2", not used in the case of the return value "3" and "4", "kcal" in the case of the return value "5", non-dimensional in the case of the return value "6", "mmHg" in the case of the return value "7", beats per minute (bpm) in the case of the return value "8". The eighth return value (shown by [7]) is the measured vital data designated by the fifth and sixth return values. The blood pressure data is, as aforementioned, returned in the form of minimal blood pressure / maximum blood pressure.

As shown in the return value section of the abnormal completion case 63A in Fig. 3, there is only one value returned when the function "getVitalData" is abnormally completed. It is only the status given by the first return value returned in the case of the normal completion (shown by [0]). When the return value is "1", it means there is no measured vital data. In case of "-1", it means a parameter error indicating that the argument shown in the argument section 62 is not in error. In case of "-2", it means a DB error showing that the measured vital data DB50 is abnormal. In case of "-3", it means an authentication error indicating that the site password "strSitePass" shown in the argument section 62 is wrong, and in case of "-4" it means another error.

The table of the above mentioned functions or the like may be delivered from the data providing side server 30 to the data utilizing side server 20 in advance. By declaring in the program executed in the data utilizing side server 20 that the function or the like is API defined in the data providing side server 30, the function or the like communicates with the data providing side server 30 and the data utilizing side server 20 can receive a predetermined response such as a supply of the measured vital data from the starting measurement date and time to the ending measurement date and time recorded in the measured vital data DB50 for the designated user. Alternatively, it is possible to receive a supply having the same contents as those of the above mentioned predetermined response, by specifying a predetermined URL of the data providing side server 30 from the data utilizing side server 20 via network 10, and by specifying, as parameters in the URL, the name of the function and the argument and the variable for receiving the return values.

The above mentioned user's ID specified by the request sent from the data utilizing side server 20 can be encrypted using a predetermined cryptography. As the predetermined cryptography, it is possible to use a desired cryptography such as SSL (Secure Sockets Layer) protocol or the public key cryptosystem.

In the above mentioned example, all the measured vital data from the measurement data and time of the starting point to the measurement data and time of the ending point was required. However, it is possible to request the measured vital data of a selected kind (measurement item such as a blood pressure or a body data percentage) of the measured vital data from the measurement data and time of the starting point to the measurement data and time of the ending point. In this case, the number of arguments becomes 5, not 4, since the argument for designating the kind of data is added. On the other hand, if the data utilizing side server 20 is in the research organization as mentioned above, the request dispatched from the data utilizing side server 20 does not include the user's identifier, and on the other hand, a function prepared in the API library 60 is not a simple retrieval function based on the user's identifier, but a combined function of a retrieval function and a statistical calculation function capable of supplying the number, the residential area, or the age distribution of users having the measured vital data value within a designated range.

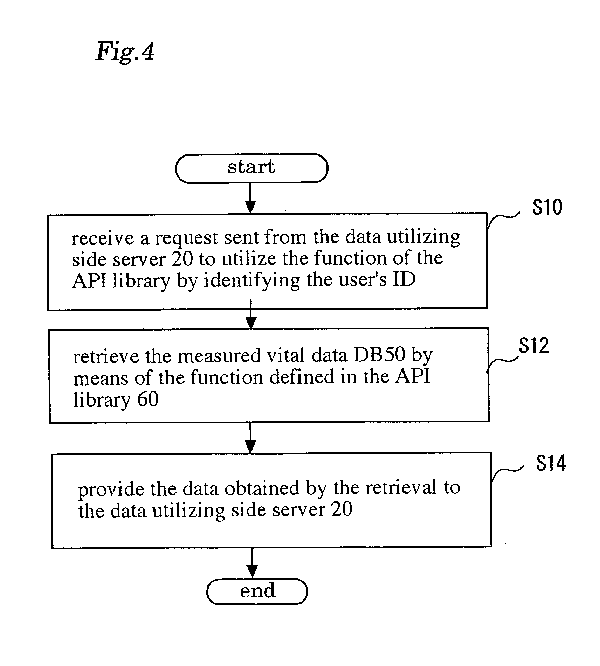

Fig. 4 shows a flowchart of the data providing program of the present invention or a flowchart of the processing in the data providing method of the present invention, executed by the data providing side server 30. As shown in Fig. 4, the data providing side server 30 receives a request which is sent from the data utilizing side server 20 to utilize the function of the API library 60 specifying the user's ID (step S10). Then, in response to this request, the data providing side server 30 retrieves the measured vital data DB50 by means of the function defined in the API library 60 and selected in accordance with that request (step S12). Authentication of the data utilizing side server 20 can be made during the execution of the function defined in the API library 60 (step S12) as mentioned above. The measured vital data obtained by the retrieval in the step S12 is provided to the data utilizing side server 20 (step S14) (all the steps S10, S12 and S14 constitute a responding step). According to the responding step mentioned above, the data providing side server 30 responds to the request which is sent from the data utilizing side server 20 and which requests to utilize the function of the API library 60 by specifying the user's ID, and can provide the data utilizing side server 20 with the measured vital data (the predetermined response) obtained by retrieving the measured vital data DB50 by using the function defined in the API library 60 and selected in accordance with the above mentioned request.

As mentioned above, according to the Embodiment 1 of the present invention, the data providing side server 30 comprises the measured vital data DB50 composed of the measured vital data records including the user's ID and the user's measured vital data, and the API library 60 for performing the retrieval and other processing for the measured vital data DB50. In response to a request sent from the data utilizing side server 20 for requesting to utilize the function of the API library 60 by specifying the user's ID, the responding section 32 of the data providing side server 30 can provide the data utilizing side server 20 with a predetermined response obtained by performing the retrieval and other processing for the measured vital data records recorded in the measured vital data DB50 by use of the function defined in the API library 60 and selected in accordance with the request concerned. When the above mentioned function is the function "getVitalData", the request for the measured vital data of the user identified by the user's ID from the measurement date and time of the starting point to the measurement date and time of the ending point, which is recorded in the measured vital data DB50, is the request to utilize the function of the API library 60 by specifying the user's ID, and the measured data thus obtained is the predetermined response to be provided to the data utilizing side server 20. Since the data utilizing side server 20 can utilize the measured vital data DB50, the data utilizing side server 20 is no longer required to construct its own unique database of measured vital data by spending a lot of time and cost. In addition, a unique website for healthcare can be constructed at a low cost and a short time because the data utilizing side server 20 can utilize the API library 60 of the data providing side server 30.

Once a user measures his or her vital data at the user's home or at a staying room in trip, or in a medical institution, the measured vital data can sent via the network to the measured vital data DB 50 and preserved and accumulated in the measured vital data DB 50. On the other hand, when the user wishes to receive a healthcare service from a healthcare service provider, the user accesses the website of the healthcare service provider having the data utilizing side server 20, and designates the kind of healthcare service in accordance with a guidance of the website and enters the user's own ID. In such a case, the data utilizing side server 20 of the healthcare service provider sends a request for measured vital data together with the user's ID via the network 10 to the data providing side server 30. The data providing side server 30 retrieves the measured vital data DB 50 so as to pick up necessary measured vital data, and sends the picked-up measured vital data to the data utilizing side server 20. The data utilizing side server 20 receives and holds the measured vital data thus sent from the data providing side server 30. The data utilizing side server 20 then treats the measured vital data of the user thus received, by means of software which was independently developed by the healthcare service provider having the data utilizing side server 20, to prepare a healthcare service requested by the user, for example, a graph presentation of the change of blood pressure in time sequence, a general living improvement advice in connection with eating habits and exercise, or an individual improvement program for each user. Therefore, the user can select a healthcare service provide depending upon the kind of healthcare service the user wants, and utilizes the healthcare service which is, the user feels, the best for the user or the most reliable. For example, if the user feels that a time sequence graph provided by a healthcare service provider "S1" is easily understandable to the user, the user can utilize the healthcare service provider "S1" in order to examine the user's measured vital data in past. On the other hand, the user can utilize a healthcare service provider "S2" in connection with the general living improvement advice in connection with eating habits, exercise, and others. Furthermore, the healthcare service providers can concentrate their capital and man-power to develop their own unique healthcare applications which utilize the measured vital data, in order to increase the number of users which use the healthcare services supplied by the healthcare service provider.

### Second Embodiment

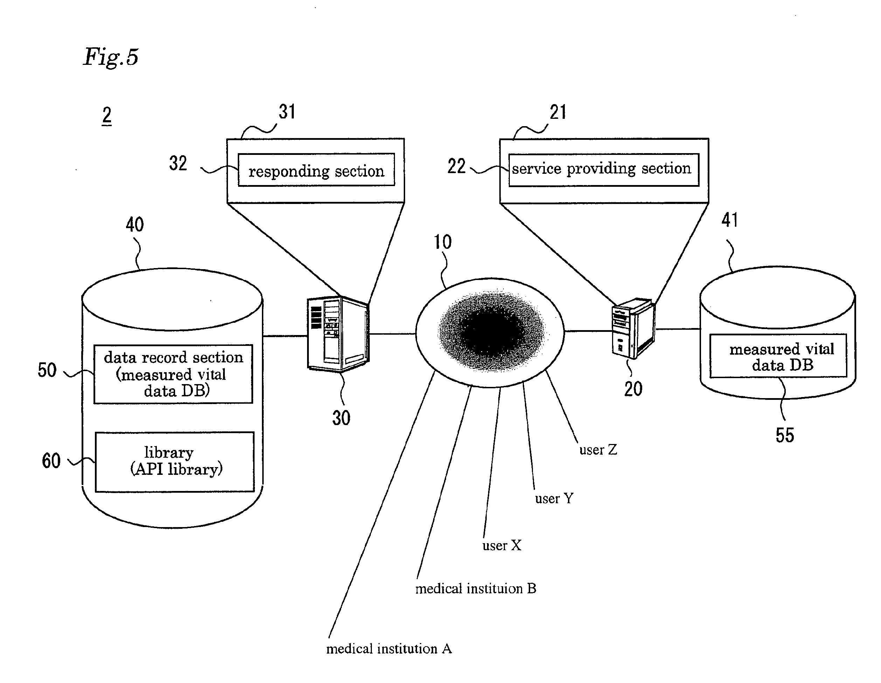

Fig. 5 shows a data providing system 2 in Embodiment 2 of the present invention. In Fig. 5, since the same Reference Numerals as those in Fig. 1 represents the same elements or functions, the description thereof will be omitted. In Fig. 5, Reference Numeral 21 is a function block indicating the function of the data utilizing side server 20, and a service providing section (service providing means) 22 is shown as its function. The service providing section 22 provides a predetermined service based on the predetermined response provided by the responding section 32. In case that the predetermined response is the measured vital data from the measurement date and time of the starting point to the measurement date and time of the ending point regarding the designated user, obtained by utilizing the function "getVitalData" described in the first embodiment, a time-sequence graphical presentation of the measured vital data may be preferable as the predetermined service. In addition, the predetermined service may be a general living improvement advice including eating habits and exercise based on the measured vital data (including an alert services based on dietetics) or an individual living improvement program for each user based on the measured vital data. The predetermined response provided by the responding section 32 is preferably recorded in a recording device 41 such asa disk storage in the data utilizing side server 20 as a measured vital data base DB55 as shown in Fig. 5.

As mentioned above, according to the Embodiment 2 of the present invention, the data utilizing side server 20 can comprise the service providing section 22 which provides a predetermined service prepared on the basis of the predetermined response provided by the responding section 32. The predetermined service mentioned above may preferably be a time-sequence graphical representation of the measured vital data, and the data utilizing side server 20 can make software for this representation in a short time and at a low cost by utilizing the function "getVitalData" explained in the first embodiment. The reason for this is that since the software of the API library can be used for the data retrieval and the primary processing of the data as mentioned above, the application software of the data utilizing side server can be specialized to the processing and the representation of the data obtained. Therefore, it is possible to develop the application software in a short time and at a low cost.

The predetermined service mentioned above may preferably be a general living improvement advice including eating habits and exercise, prepared on the basis of the measured vital data, and the data utilizing side server 20 can prepare the contents of the advice in a short time and at a low cost by utilizing the function "getVitalData" explained in the first embodiment. Thus, in addition to the advantages obtained in the first embodiment, the data utilizing side server 20 can construct in a short time and at low cost a unique website for healthcare which comprises software and contents for performing the predetermined service. Namely, the data providing side server 30 comprising the measured vital data DB50 and the data utilizing side server 20 comprising the software and the contents for the predetermined service can be developed independently of each other. Consequently, an administering entity (company, organization, etc.) of the data providing side server 30 and an administering entity of the data utilizing side server 20 may be a different capital or may belong to different business sectors, so that cooperative relationship between enterprises in various business areas can be developed increasingly.

### Third Embodiment

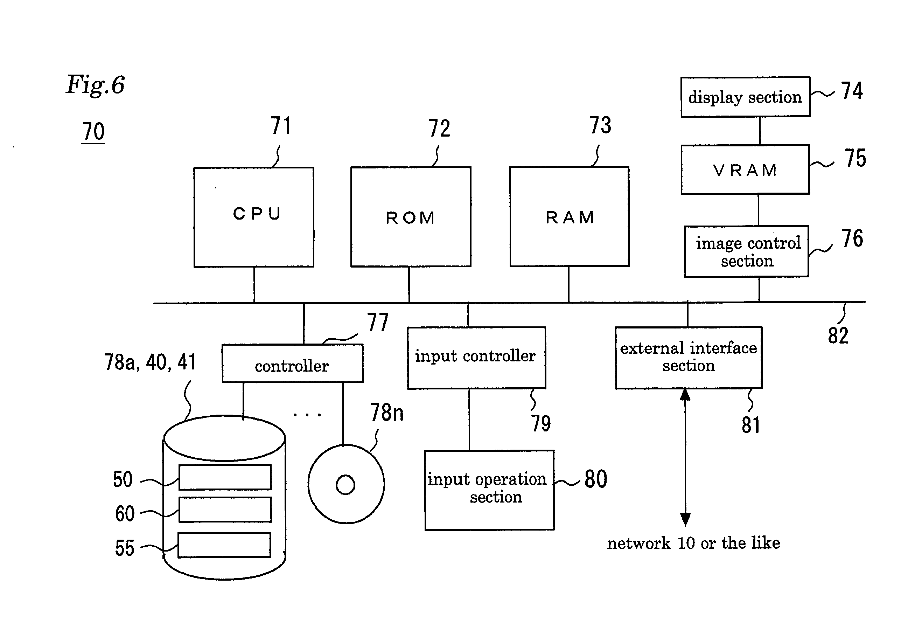

Fig. 6 is a block diagram of a computer internal circuit 70 of the data providing side server 30 or the data utilizing side server 20, which executes the computer programs of the present invention to implement the above embodiments. As shown in Fig. 6, a CPU 71, a ROM 72, a RAM 73, an image control section 76, an controller 77, an input controller 79 and an external interface (I/F) section 81 are connected to a bus 82. In Fig. 6, the above computer programs of the present invention are recorded in a storage medium (including removable storage medium) such as the ROM 72, a disk storage 78a, a storage device 40 or 41, or a DVD or CD-ROM driver 78n or the like. The above mentioned measured vital data DB 50 or 55 and the API library 60 are also recorded in the disk storage 78a or the like. The computer programs of the present invention are loaded from the ROM. 72 via the bus 82 or from the storage medium such as the disk storage 78a or the DVD driver 78n or the like via the controller 77 and the bus 82 to the RAM 73. The input control section 79 is connected to an input operation section 80 such as a mouse and a numeric keyboard in order to control the inputs or the like. An image memory VRAM 75 has a capacity of data corresponding to at least one frame of the display section 74 of the data providing side server 30 or the like, and the image control section 76 has a function to convert data from the VRAM 75 into image data and to send it to the display section 74. The external interface section 81 has an input and output interface function for interfacing with the network 10 or the like.

As described above, the object of the present invention can be achieved by means of the CPU 71 executing the computer programs of the present invention described above. The computer programs can be supplied to the computer CPU 71 in the form of a recording medium such as DVD 78n or the like. Therefore, the recording medium such as DVD 78n or the like having the computer programs recorded thereon also constitutes the present invention. As a recording medium having the computer program recorded thereon other than the above described recording medium, for example, a memory card, a memory stick, a laser disk, FD or the like can be also used.

### Industrial Applicability

As utilization examples of the data providing system or the like of the present invention, it can be applied to the field of health guidance or preventive medical care.

## Claims

1. A measured vital data preserving and providing system for healthcare, comprising a data providing side server and a data utilizing side server, which are connected via a network, the data utilizing side server being configured to respond to a user's request so as to provide a service obtained by utilizing the data preserved in the data providing side server,
wherein the data providing side server comprises:
a data record section for recording data records including an identifier concerning a user and data associated with said identifier,
a library of a plurality of functions capable of processing said data records recorded in said data record section, and
a responding means for responding to a request which is sent from said data utilizing side server and which identifies a selected identifier and requests to utilize a function selected from the plurality of functions of the library, so as to provide to said data utilizing side server a predetermined response obtained by processing said data records recorded in said data record section by use of said selected function of the library.

2. A measured vital data preserving and providing system for healthcare, according to Claim 1, wherein said data utilizing side server includes a service providing means responding to a request from said user to provide a predetermined service based on said predetermined response supplied from said responding means.

3. A measured vital data preserving and providing system for healthcare, according to Claim 1, wherein said identifiers included in said data records are user's identifiers, and the data included in said data records is a vital data of users measured by a vital data measuring device.

4. A measured vital data preserving and providing system for healthcare, according to Claim 1, wherein at least one function of the plurality of functions of said library is a function for retrieving said data records preserved in said data record section, with reference to said identifier designated in said request sent from said data utilizing side server.

5. A measured vital data preserving and providing system for healthcare, according to Claim 1, wherein said identifier designated in said request sent from said data utilizing side server may be an identifier enciphered in a predetermined manner.

6. A data providing program for a data providing side server to provide data via a network, said data providing side server being connected to a data utilizing side server via said network, said data providing side server comprising a data record section for recording data records including an identifier concerning a user and data associated with said identifier, and a library of a plurality of functions capable of processing said data records recorded in said data record section,
the data providing program enabling a computer of said data providing side server to function as a responding means for responding to a request which is sent from said data utilizing side server and which identifies a selected identifier and requests to utilize a function selected from the plurality of functions of said library, so as to provide to said data utilizing side server a predetermined response obtained by processing the said records recorded in said data record section by use of the selected function of said library.

7. A data providing program according to Claim 6, wherein said identifiers included said the data records are user's identifiers, and said data included in said data records is a vital data of users measured by a vital data measuring device.

8. A data providing program according to Claim 6, wherein the data providing program can decipher the identifier enciphered in a predetermined manner, designated in the request sent from said data utilizing side server.

9. A computer-readable recording medium recording said data providing program defined in Claim 6.

10. A data providing method for providing data from a data providing side server to a data utilizing side server via a network, the data providing side server comprising a data record section for recording data records including an identifier concerning a user and data associated with said identifier, and a library of a plurality of functions capable of processing said data records recorded in said data record section, the data utilizing side server responding to a user's request so as to provide a service obtained by utilizing the data preserved in said data providing side server, the method including the steps of:
causing said data utilizing side server to generate a request which identifies a selected identifier and requests to utilize a function selected from said plurality of functions of said library, to said data providing side server. and
causing said data providing side server to provide to said data utilizing side server via the network a predetermined response obtained by processing said data records recorded in said data record section and designated by the identifier included in said request, by use of the function selected by said request, of said library of said data providing side server
